# EUROPEAN PATENT SPECIFICATION

(11) **EP 2 463 648 B1**
(45) Date of publication and mention of the grant of the patent: **01.11.2017**
(21) Application number: 11193248.9
(22) Date of filing: 13.12.2011
(51) Int. Cl.: G01N 27/407, G01N 33/00

(54) **Multigas sensor**
Multigassensor
Capteur multigaz

(30) Priority: 13.12.2010 JP 2010276707
(43) Date of publication of application: 13.06.2012
(73) Proprietor: NGK Spark Plug Co., Ltd., Nagoya-shi Aichi 467-8525 (JP)
(72) Inventor: Sugaya, Satoshi, Nagoya Aichi (JP); Kakimoto, Shiro, Nagoya Aichi (JP); Nakano, Yoshihiro, Nagoya Aichi (JP); Kojima, Takio, Nagoya Aichi (JP)
(74) Representative: Grünecker Patent- und Rechtsanwälte PartG mbB

(56) References cited:
- JP-A- 2010 038 806
- US-A1- 2007 080 074
- US-A1- 2008 223 110
- US-A1- 2010 242 574

## Description

### BACKGROUND OF THE INVENTION

### 1. Field of the Invention

The present invention relates to a multigas sensor suited for detecting nitrogen oxide concentration and ammonia concentration in a gas-to-be-measured.

### 2. Description of the Related Art

Known gas sensors for measuring the concentration of a particular gas in a gas-to-be-measured, such as automotive exhaust gas, include an NOₓ sensor which detects NOₓ concentration in the gas-to-be-measured while using a solid electrolyte body, and an ammonia sensor which detects ammonia concentration in the gas-to-be-measured by utilizing a change in impedance or electromotive force between paired electrodes.

Furthermore, a proposed technique for simultaneously measuring NOₓ concentration and ammonia concentration in the gas-to-be-measured has a step in which the gas-to-be-measured is brought into contact with an NH₃ strong oxidizing catalyst for converting ammonia to NOₓ, thereby measuring total NOₓ concentration, and a step in which the gas-to-be-measured is brought into contact with an NH₃ weak oxidizing catalyst for converting a portion of ammonia to NOₓ, thereby measuring NOₓ concentration. From the two values detected in these steps, NOₓ concentration and ammonia concentration in the gas-to-be-measured are calculated (refer to Patent Document 1).

However, the technique described in Patent Document 1 utilizes a difference in catalytic capability for measurement and thus involves a problem in that catalytic capability varies with a variation of environmental measurement conditions (temperature, flow rate, pressure, etc.), resulting in inaccurate measurement. Furthermore, when concentrations of a plurality of gases are measured by use of respectively separate sensors, since the sensors are not disposed at the same position, gas concentration, temperature distribution, etc., differ from sensor to sensor, and also time lag in measurement arises among the sensors. These factors may affect measurement.

Thus, a multigas sensor has been proposed in which a single gas sensor element has an NOₓ sensor portion and an ammonia sensor portion. By employing such a configuration, the sensor portions are exposed to the same gas concentration, the same temperature distribution, etc., while time lag in measurement between the sensor portions is restrained. Therefore, the multigas sensor can measure NOₓ concentration and ammonia concentration with high accuracy (refer to Patent Document 2).
[Patent Document 1] Japanese Patent Application Laid-Open (*kokai*) No. 2001-133447
[Patent Document 2] Japanese Patent Application Laid-Open (*kokai*) No. 2010-38806

### 3. Problems to be Solved by the Invention

The gas sensor element of the multigas sensor as described in Patent Document 2 usually has the NOₓ sensor portion and the ammonia sensor portion at its front end portion. Also, the gas sensor element is held in a tubular metallic shell while its front end portion projects from the front end of the metallic shell. Furthermore, a protector fixed to a front end portion of the metallic shell covers the front end portion of the gas sensor element. The protector restrains direct adhesion of water in an exhaust pipe to the gas sensor element. The protector assumes a closed-bottomed tubular shape and has gas introduction holes formed in its side wall for allowing introduction of a gas-to-be-measured into the interior thereof and a gas discharge hole formed in its front end wall for allowing discharge of the gas-to-be-measured from the interior thereof.

Meanwhile, in order to appropriately detect NOₓ concentration in the gas-to-be-measured, the temperature of the NOₓ sensor portion is controlled to a high temperature of about 750°C. Thus, when the gas-to-be-measured which has been introduced into the interior of the protector through the gas introduction holes passes the vicinity of the NOₓ sensor portion (more specifically, a gap between the protector and the gas sensor element in the vicinity of the NOₓ sensor portion) before reaching the ammonia sensor portion, the controlled high temperature of the NOₓ sensor portion causes thermal decomposition of ammonia contained in the gas-to-be-measured. Accordingly, ammonia concentration in the gas-to-be-measured which has reached the ammonia sensor portion may differ from ammonia concentration in the actual gas-to-be-measured. Thus, the ammonia sensor portion may fail to accurately detect ammonia concentration in the gas-to-be-measured.

### SUMMARY OF THE INVENTION

It is therefore an object of the present invention to provide a multigas sensor which can measure NOₓ concentration and ammonia concentration by means of a single gas sensor element and which prevents a gas-to-be-measured, which has passed by a high-temperature NOₓ sensor portion and has undergone associated thermal decomposition of ammonia, from reaching an ammonia sensor portion, for accurate measurement of ammonia concentration.

The above object of the invention have been achieved by providing a multigas sensor which comprises a gas sensor element, a tubular metallic shell, and a closed-bottomed tubular protector. The gas sensor element extends in an axial direction and has an NOₓ sensor portion and an ammonia sensor portion at its front end portion. The ammonia sensor portion is exposed from an outer surface of the front end portion of the gas sensor element. The metallic shell holds the gas sensor element such that a front end portion of the gas sensor element projects from its front end. The protector is fixed to a front end portion of the metallic shell and covers the front end portion of the gas sensor element. The protector has a gas introduction hole formed in its side wall, and a gas discharge hole formed in its front end wall adapted to discharge the gas-to-be-measured. In the multigas sensor, at least a subportion of the ammonia sensor portion is disposed within a positional range along the axial direction between the gas introduction hole and the gas discharge hole. Also, a shortest distance between the gas introduction hole and the ammonia sensor portion is shorter than a shortest distance between the gas introduction hole and the gas diffusion hole.

The NOₓ sensor portion preferably includes a first pumping cell and a second pumping cell. The first pumping cell has a first solid electrolyte body, and a pair of first electrodes disposed on the first solid electrolyte body so as to be located inside and outside, respectively, of a first measuring chamber, and is adapted to pump oxygen out of or into a gas-to-be-measured that has been introduced into the first measuring chamber via a gas diffusion hole. The second pumping cell has a second solid electrolyte body, and a pair of second electrodes disposed on the second solid electrolyte body so as to be located inside and outside, respectively, of an NOₓ measuring chamber in communication with the first measuring chamber. A second pumping current flows between the paired second electrodes of the second pumping cell in accordance with NOₓ concentration in the gas-to-be-measured, whose oxygen concentration has been adjusted in the first measuring chamber and which has flowed into the NOₓ measuring chamber.

The ammonia sensor portion is preferably configured such that at least a pair of electrodes is disposed on a solid electrolyte body, and is adapted to output an ammonia concentration output.

According to the thus-configured multigas sensor, the shortest distance d₁ between the gas introduction hole and the ammonia sensor portion is smaller than the shortest distance d₂ between the gas introduction hole and the gas diffusion hole (d₁ < d₂). Accordingly, the gas-to-be-measured first comes into contact with the ammonia sensor portion which is closer to the gas introduction hole than the gas diffusion hole. Thus, the gas-to-be-measured which has been introduced into the protector through the gas introduction hole does not pass in the vicinity of the NOₓ sensor portion whose temperature is raised, before reaching the ammonia sensor portion. Therefore, the fresh gas-to-be-measured before undergoing thermal decomposition of ammonia (i.e., the gas-to-be-measured in the same condition as that of the gas-to-be-measured which passes through the gas introduction hole) can reach the ammonia sensor portion, thereby improving accuracy in measuring ammonia concentration. Also, since the temperature of the NOₓ sensor portion can be controlled to be sufficiently high, accuracy in measuring NOₓ concentration also improves.

Also, at least a subportion of the ammonia sensor portion is disposed within a positional range along the axial direction between the gas introduction hole and the gas discharge hole. Thus, the ammonia sensor portion is present on a gas-to-be-measured flow path from the gas introduction hole to the gas discharge hole. Therefore, the ammonia sensor portion can measure ammonia concentration in such a condition that sufficient gas-to-be-measured is supplied (diffused) thereto. Accordingly, even when the flow rate of the gas-to-be-measured varies, the ammonia sensor portion can stably measure ammonia concentration. As a result, the ammonia sensor portion exhibits high responsiveness in ammonia concentration detection.

Meanwhile, a flow of the gas-to-be-measured from the gas introduction hole to the gas discharge hole is generated by the following mechanism. When the multigas sensor is mounted to an exhaust pipe or the like, the flow rate of the gas-to-be-measured in the exhaust pipe or the like increases in the vicinity of the gas discharge hole formed in the front end of the multigas sensor. Accordingly, pressure in the protector is lower in the discharge hole than in the gas introduction hole.

Furthermore, the multigas sensor of the present invention may be configured as follows: the gas diffusion hole is disposed within a positional range along the axial direction between the gas discharge hole and the ammonia sensor portion.

By employing the above configuration, the gas diffusion hole is present on a gas-to-be-measured flow path from the gas introduction hole to the gas discharge hole. Thus, the NOₓ sensor portion can measure NOₓ concentration in such a condition that sufficient gas-to-be-measured is supplied (diffused) thereto, and the NOₓ sensor portion exhibits high responsiveness in NOₓ concentration detection.

Furthermore, preferably, the multigas sensor of the present invention is configured as follows: a shortest distance between the ammonia sensor portion and an inner surface of the side wall of the protector is shorter than a shortest distance between the gas sensor element excluding the ammonia sensor portion and the inner surface of the side wall of the protector.

By employing the above configuration, by means of the venturi effect, the flow rate of the gas-to-be-measured in a gap between the protector and the gas sensor element as measured in the vicinity of the ammonia sensor portion is higher than the flow rate of the gas-to-be-measured in the gap between the protector and the gas sensor element as measured in other regions. Thus, sufficient gas-to-be-measured is supplied (diffused) to the ammonia sensor portion, thereby further improving accuracy in measuring ammonia concentration.

In order to implement the above configuration, a portion of the side wall of the protector where a distance between the side wall and the ammonia sensor portion is shortest may project radially inward.

By employing the above configuration, the aforementioned venturi effect can be readily implemented.

Furthermore, the multigas sensor of the present invention may be configured as follows: the NOₓ sensor portion and the ammonia sensor portion overlap each other in the axial direction.

By employing the above configuration, in contrast to the case where the NOₓ sensor portion and the ammonia sensor portion do not overlap each other (i.e., where the sensor portions are located away from each other) in the axial direction, the sensor portions can be exposed to substantially the same gas-to-be-measured. Thus, NOₓ concentration and ammonia concentration can be measured with higher accuracy.

The shortest distance between the ammonia sensor portion and the gas introduction hole may be shorter than a shortest distance between the ammonia sensor portion and the gas discharge hole.

By employing the above configuration, the gas-to-be-measured in a condition before diffusion toward the gas discharge hole and similar to the condition of the gas-to-be-measured which passes through the gas introduction hole can reach the ammonia sensor portion. Thus, accuracy in measuring ammonia concentration is improved.

### EFFECT OF THE INVENTION

The present invention enables measurement of NOₓ concentration and ammonia concentration by means of a single gas sensor element. More particularly, the present invention prevents a gas-to-be-measured, which has passed near by a high-temperature NOₓ sensor portion so as to have undergone associated thermal decomposition of ammonia, from reaching an ammonia sensor portion. As a result, the present invention enables accurate measurement of ammonia concentration.

### BRIEF DESCRIPTION OF THE DRAWINGS

FIG. 1 is a sectional view of a multigas sensor according to an embodiment of the present invention taken along the longitudinal direction of the multigas sensor.
FIG. 2 is a block diagram showing the configuration of the multigas sensor according to the embodiment of the present invention and a controller.
FIG. 3 is a perspective view showing the schematic configuration of an NOₓ sensor portion.
FIG. 4 is an exploded perspective view showing the configuration of an ammonia sensor portion.
FIG. 5 is a sectional view showing positional relations of holes of a protector with the NOₓ sensor portion and the ammonia sensor portion.
FIGS. 6A to 6D are sectional views showing modifications of the multigas sensor.
FIGS. 7A to 7D are sectional views showing other modifications of the multigas sensor.
FIGS. 8A to 8C are sectional views showing multigas sensors of the Comparative Examples.
FIG. 9 is a graph showing accuracy in detection of ammonia concentration (ammonia concentration output at an NH₃ content of 20 ppm and 50 ppm) of the Examples and Comparative Examples.
FIG. 10 is a pair of graphs showing the flow rate dependency of ammonia concentration detection of Examples 1 and 7.
FIG. 11 is a pair of graphs showing flow rate dependency of ammonia concentration detection of Example 8 and Comparative Example 1.
FIG. 12 is a pair of graphs showing the responsiveness in ammonia detection of Example 1.
FIG. 13 is a pair of graphs showing the responsiveness in ammonia detection of Comparative Example 1.

### DESCRIPTION OF REFERENCE NUMERALS

Reference numerals used to identify various structural elements in the drawings include the following:
2a: first solid electrolyte body
2b, 2c: first electrode (inner first pumping electrode, outer first pumping electrode)
2: first pumping cell
4a: second solid electrolyte body
4b, 4c: second electrode (inner second pumping electrode, second pumping counter electrode)
4: second pumping cell
8a: gas diffusion hole
25: (ammonia sensor portion) solid electrolyte body
30: NOₓ sensor portion
42: ammonia sensor portion
42a: a pair of electrodes
44A, 44B: diffusion layer
100A: gas sensor element
105: front end portion of gas sensor element
138: metallic shell
141, 142, 143: protector
142a: (outer) gas introduction hole
143a: (inner) gas introduction hole
143b: (inner) gas discharge hole
143d: (inner) side wall (of protector)
143t: front end wall (of protector)
143s: inner surface of side wall (of protector)
143p: diameter-reduced portion (of protector)
200A: multigas sensor
d₁: shortest distance between gas introduction hole and ammonia sensor portion
d₂: shortest distance between gas introduction hole and diffusion hole
d₃: shortest distance between ammonia sensor portion and inner surface of side wall of protector
d₄: shortest distance between gas sensor element excluding ammonia sensor portion and inner surface of side wall of protector
d₅: shortest distance between ammonia sensor portion and gas discharge hole
S1: first measuring chamber
S2: NOₓ measuring chamber
R₁: positional range along axial direction between gas introduction hole and gas discharge hole
R₂: positional range along axial direction between gas discharge hole and ammonia sensor portion
O: axial direction

### DETAILED DESCRIPTION OF THE PREFERRED EMBODIMENTS

An embodiment of the present invention will next be described by reference to the drawings. However, the present invention should not be construed as being limited thereto.

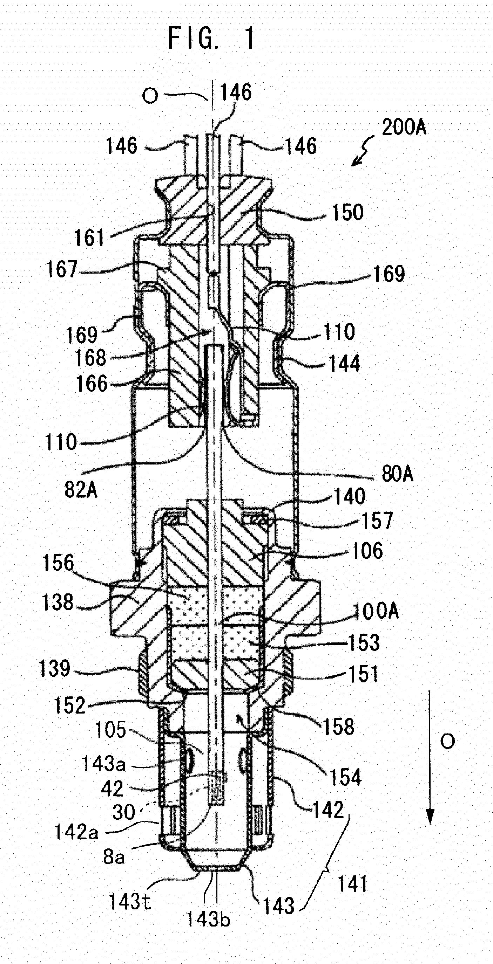

FIG. 1 is a sectional view of a multigas sensor 200A according to the embodiment of the present invention taken along the longitudinal direction (axial direction O) of the multigas sensor 200A. The multigas sensor 200A is an assembly having a gas sensor element 100A for detecting ammonia concentration and NOₓ concentration. The multigas sensor 200A includes a plate-like gas sensor element 100A extending in the axial direction O; a tubular metallic shell 138 having a threaded portion 139 formed on its outer surface and adapted to be fixed to an exhaust pipe; a closed-bottomed cylindrical protector 141 fixed to a front end portion of the metallic shell 138; a tubular ceramic sleeve 106 disposed so as to radially surround the gas sensor element 100A; an insulation contact member 166 having a contact insertion hole 168 extending therethrough in the axial direction O, and disposed so that the wall surface of the contact insertion hole 168 radially surrounds a rear end portion of the gas sensor element 100A; and a plurality of connection terminals 110 (in FIG. 1, only two of them are shown) disposed between the gas sensor element 100A and the insulation contact member 166.

Notably, the lower side of FIG. 1 (the side toward the gas sensor element 100A) with respect to the axial direction O is referred to as the "front side," and the upper side of FIG. 1 (the side toward a grommet 150) is referred to as the "rear side."

Although described in detail below, the gas sensor element 100A has an NOₓ sensor portion 30 and an ammonia sensor portion 42 at its front end portion 105, and the ammonia sensor portion 42 is exposed from the outer surface of the gas sensor element 100A. Also, the gas sensor element 100A has gas diffusion holes 8a opening at its respective side surfaces and adapted to introduce a gas-to-be-measured into the interior of the NOₓ sensor portion 30.

The metallic shell 138 assumes a substantially tubular shape and has a through hole 154 extending therethrough in the axial direction O and a ledge 152 projecting radially inward in the through hole 154. The metallic shell 138 holds the gas sensor element 100A in the through hole 154 in the following condition: the front end portion 105 of the gas sensor element 100A is disposed externally of the front end of the through hole 154, and electrode terminal portions 80A and 82A are disposed externally of the rear end of the through hole 154. Furthermore, the ledge 152 assumes the form of a radially inward taper surface inclined with respect to a plane perpendicular to the axial direction O.

An annular ceramic holder 151, powder filler layers 153 and 156 (hereinafter, may be referred to as the talc rings 153 and 156), and the above-mentioned ceramic sleeve 106 are stacked in this order from the front side to the rear side within the through hole 154 of the metallic shell 138 so as to radially surround the gas sensor element 100A. Also, a crimp packing 157 is disposed between the ceramic sleeve 106 and a rear end portion 140 of the metallic shell 138. A metal holder 158 is disposed between the ceramic holder 151 and the ledge 152 of the metallic shell 138 for holding the talc ring 153 and the ceramic holder 151 and for maintaining gastightness. The rear end portion 140 of the metallic shell 138 is crimped so as to press frontward the ceramic sleeve 106 via the crimp packing 157.

Meanwhile, as shown in FIG. 1, the closed-bottomed cylindrical protector 141 made of metal (e.g., stainless steel) is attached, by welding or the like, to the outer circumference of a front end portion (in FIG. 1, a lower end portion) of the metallic shell 138. The protector 141 covers the front end portion 105 of the gas sensor element 100A and has a plurality of outer gas introduction holes 142a, a plurality of inner gas introduction holes 143a, and an inner gas discharge hole 143b. Although the protector 141 is described in detail below, in the present embodiment, the protector 141 has a dual structure in which a tubular outer protector 142 is disposed radially outward of a closed-bottomed tubular inner protector 143 while being spaced apart from the inner protector 143, and a front end wall 143t of the inner protector 143 projects frontward from an opening formed in a bottom wall 142t of the outer protector 142.

Meanwhile, a tubular sheath 144 is fixed to the outer circumference of a rear end portion of the metallic shell 138. A grommet 150 is disposed in a rear-end (in FIG. 1, an upper-end) opening portion of the tubular sheath 144. The grommet 150 has lead-wire insertion holes 161 through which a plurality of lead wires 146 (in FIG. 1, only three lead wires are shown) are inserted respectively for electrical connection to the electrode terminal portions 80A and 82A of the gas sensor element 100A. For simplification, FIG. 1 representatively shows the electrode terminal portions 80A and 82A on the front and back surfaces of the gas sensor element 100A. In actuality, a plurality of electrode terminal portions are formed according to the number of electrodes or the like of the NOₓ sensor portion 30 and the ammonia sensor portion 42, which will be described below.

The insulation contact member 166 is disposed at a position corresponding to a rear end portion (in FIG. 1, an upper end portion) of the gas sensor element 100A which projects from the rear end portion 140 of the metallic shell 138. The insulation contact member 166 is disposed around the electrode terminal portions 80A and 82A formed on the front and back surfaces of the rear end portion of the gas sensor element 100A. The insulation contact member 166 assumes a tubular shape and has the contact insertion hole 168 extending therethrough in the axial direction O, as well as a flange portion 167 projecting radially outward from the outer surface thereof. The insulation contact member 166 is disposed within the tubular sheath 144 by means of the flange portion 167 coming into contact with the tubular sheath 144 via a holding member 169. Electrical connection is established between the connection terminals 110 on a side toward the insulation contact member 166 and the electrode terminal portions 80A and 82A of the gas sensor element 100A, thereby establishing electrical communication with an external system via the lead wires 146.

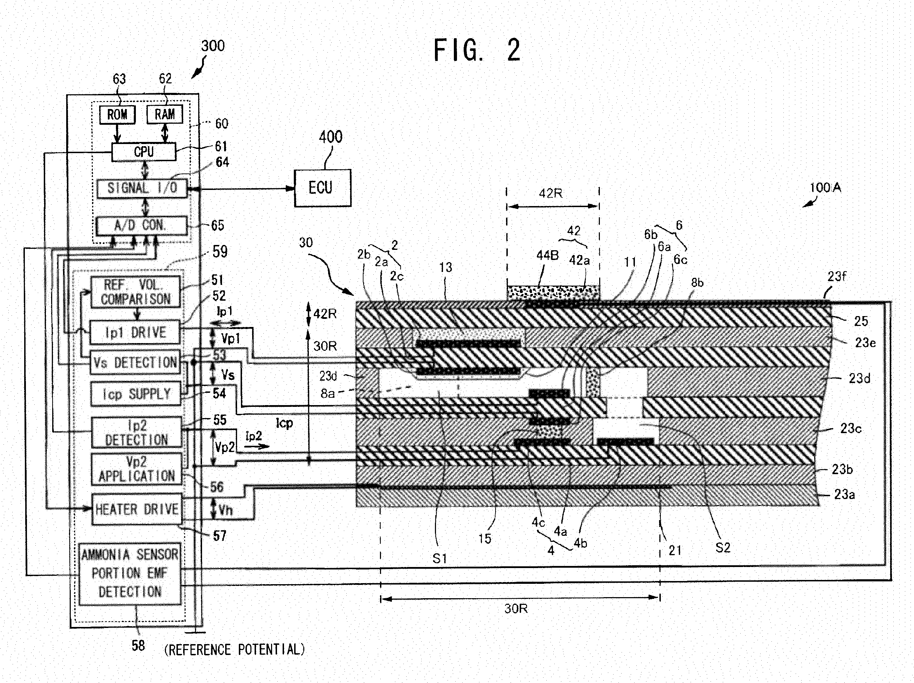

FIG. 2 is a block diagram showing the configuration of a controller 300 according to the embodiment of the present invention and the gas sensor element 100A connected to the controller 300. For convenience of description, FIG. 2 shows only the longitudinal section of the gas sensor element 100A of the multigas sensor 200A.

The multigas sensor 200A (gas sensor element 100A) and the controller 300 are mounted in an unillustrated vehicle having an internal combustion engine (hereinafter, also referred to as an engine). The controller 300 is electrically connected to a vehicular control unit (hereinafter, referred to as the "ECU") 400. Ends of the lead wires 146 extending from the multigas sensor 200A are connected to respective connectors, which, in turn, are electrically connected to corresponding connectors of the controller 300.

The ECU 400 receives data on ammonia concentration and NOₓ concentration in the exhaust gas, which concentrations are calculated in the controller 300. On the basis of the received data, the ECU 400 controls the operating conditions of the engine and performs various processes, such as a process of cleaning accumulated NOₓ off a catalyst.

Next, the configuration of the gas sensor element 100A is described. The gas sensor element 100A includes the NOₓ sensor portion 30 having a configuration similar to that of a publicly known NOₓ sensor, and the ammonia sensor portion 42 having a configuration similar to that of a publicly known ammonia sensor. As described in detail below, the ammonia sensor portion 42 is exposed from the outer surface of the gas sensor element 100A.

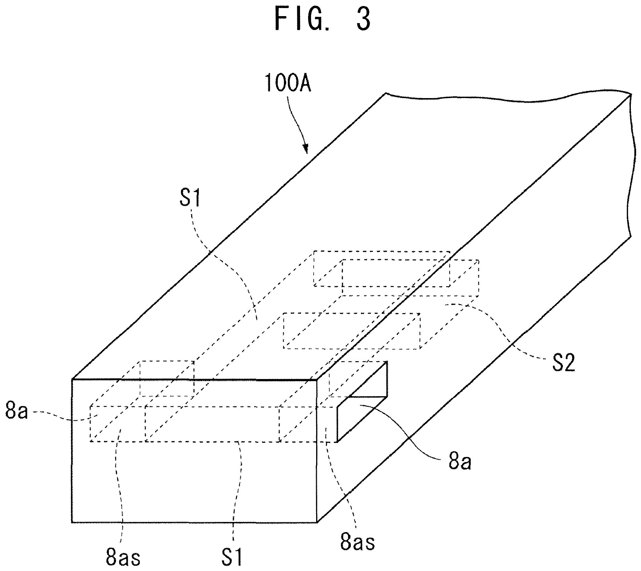

First, the NOₓ sensor portion 30 has a structure in which an insulation layer 23f, an ammonia sensor portion solid electrolyte body 25, an insulation layer 23e, a first solid electrolyte body 2a, an insulation layer 23d, a third solid electrolyte body 6a, an insulation layer 23c, a second solid electrolyte body 4a, and insulation layers 23b and 23a are laminated together in this order. A first measuring chamber S1 is formed between the first solid electrolyte body 2a and the third solid electrolyte body 6a. The first measuring chamber S1 communicates with an ambient atmosphere through communication portions provided in side regions of a front end portion of the gas sensor element 100A and through the rectangular diffusion holes 8a formed in the side surfaces of the gas sensor element 100A (see FIG. 3). A first diffusion resistor 8as is disposed in each of the communication portions extending between the first measuring chamber S1 and the gas diffusion holes 8a, for introducing the gas-to-be-measured into the first measuring chamber S1 through a predetermined diffusion resistance from the ambient atmosphere (from the side surfaces of the gas sensor element 100A).

A second diffusion resistor 8b is disposed at the rear end of the first measuring chamber S1. A second measuring chamber (which corresponds to the "NOₓ measuring chamber" in the present invention) S2 is formed rearward of the first measuring chamber S1 and communicates with the first measuring chamber S1 via the second diffusion resistor 8b. The second measuring chamber S2 is formed between the first solid electrolyte body 2a and the second solid electrolyte body 4a while extending through the third solid electrolyte body 6a.

A first pumping cell 2 includes the first solid electrolyte body 2a which predominantly contains oxygen ion conductive zirconia, an inner first pumping electrode 2b, and an outer first pumping electrode 2c (the electrodes 2b and 2c correspond to the "first electrodes" in the invention), the electrodes 2b and 2c being paired and disposed with the first solid electrolyte body 2a sandwiched therebetween. The inner first pumping electrode 2b faces the first measuring chamber S1. The inner first pumping electrode 2b and the outer first pumping electrode 2c predominantly contain platinum. The inner first pumping electrode 2b is covered with a protection layer 11 formed of a porous body.

A portion of the insulation layer 23e which corresponds to the upper surface of the outer first pumping electrode 2c is cut out. The resultant cutout space is filled with a porous body 13 for allowing the outer first pumping electrode 2c to communicate with the ambient atmosphere, thereby enabling inflow and outflow of gas (oxygen).

An oxygen concentration detection cell 6 includes a third solid electrolyte body 6a which predominantly contains zirconia, and a detection electrode 6b and a reference electrode 6c which are disposed with the third solid electrolyte body 6a sandwiched therebetween. The detection electrode 6b faces the first measuring chamber S1 at a position located downstream of the inner first pumping electrode 2b. The detection electrode 6b and the reference electrode 6c predominantly contain platinum.

A portion of the insulation layer 23c is cut out so that the reference electrode 6c in contact with the third solid electrolyte body 6a is disposed in the resultant cutout space, and the cutout space is filled with a porous body, thereby forming a reference oxygen chamber 15. By use of an Icp supply circuit 54, a very weak constant current is supplied to the oxygen concentration detection cell 6 beforehand. By this procedure, oxygen is transported from the first measuring chamber S1 to the reference oxygen chamber 15, thereby establishing an oxygen reference.

A second pumping cell 4 includes the second solid electrolyte body 4a which predominantly contains zirconia, an inner second pumping electrode 4b disposed on the second solid electrolyte body 4a and facing the second measuring chamber S2, and a second pumping counter electrode 4c (the electrodes 4b and 4c correspond to the "second electrodes" in the invention). The inner second pumping electrode 4b and the second pumping counter electrode 4c predominantly contain platinum.

The second pumping counter electrode 4c is disposed on the second solid electrolyte body 4a in the cutout space of the insulation layer 23c and faces the reference electrode 6c and the reference oxygen chamber 15.

The inner first pumping electrode 2b, the detection electrode 6b, and the inner second pumping electrode 4b are connected to a reference potential.

In order to specify positional relations, which will be described below, between the NOₓ sensor portion 30 and the inner gas introduction holes 143a and the inner gas discharge hole 143b of the protector 141, a region which encompasses the outlines of the first pumping cell 2, the second pumping cell 4, the oxygen concentration detection cell 6, the first measuring chamber S1, the second measuring chamber S2, and the gas diffusion holes 8a communicating with the first measuring chamber S1 is considered to be the NOₓ sensor portion 30 (the domain of the NOₓ sensor portion 30). For example, a region 30R ranging from the front end of the first measuring chamber S 1 to the rear end of the second measuring chamber S2 with respect to the axial direction O and ranging from the protection layer 13 (the insulation layer 23e) to the second solid electrolyte body 4a with respect to the lamination direction perpendicular to the axial direction O is the domain of the NOₓ sensor portion 30 (FIG. 2 shows the ranges of the region 30R with respect to the axial direction O and the direction perpendicular to the axial direction O, respectively).

A porous component, such as the protection layer 13, is considered to fall in the region occupied by the NOₓ sensor portion 30. This is for the following reason: since the gas-to-be-measured stagnates in a porous component because of resistance to flow, a flow of the gas-to-be-measured in the porous component differs from a free flow of the gas-to-be-measured in a region outside the NOₓ sensor portion 30.

An elongated plate-like heater 21 is embedded between the insulation layers 23b and 23a while extending in the longitudinal direction of the gas sensor element 100A. The heater 21 heats the NOₓ sensor portion 30 and the ammonia sensor portion 42 to respectively predetermined temperatures (e.g., 750°C for the first pumping cell 2 and 600°C for the second pumping cell 4 and the ammonia sensor portion 42), thereby enhancing oxygen ion conductivity for stable operation.

The insulation layers 23a, 23b, 23c, 23d, 23e and 23f predominantly contain alumina. The first diffusion resistors 8as and the second diffusion resistor 8b are formed of a porous substance, such as alumina. The heater 21 is formed of platinum or the like.

FIG. 3 is a perspective view showing the schematic configuration of the NOₓ sensor portion 30. The first measuring chamber S1 communicates with an ambient atmosphere through communication portions provided in side regions of a front end portion of the gas sensor element 100A and through the rectangular diffusion holes 8a formed in the side surfaces of the gas sensor element 100A. The first diffusion resistors 8as are disposed in the respective communication portions. The second measuring chamber S2 is formed rearward of the first measuring chamber S 1.

Referring back to FIG. 2, the ammonia sensor portion 42 is formed on the insulation layer 23f, which serves as the outer surface of the NOₓ sensor portion 30. However, a rectangular portion of the insulation layer 23f is cut out, whereby an associated portion of the ammonia sensor portion solid electrolyte 25 is exposed. A pair of electrodes 42a of the ammonia sensor portion 42 is formed on the exposed portion of the ammonia sensor solid electrolyte body 25. On the basis of a change in electromotive force between the paired electrodes 42a, ammonia concentration in the gas-to-be-measured is detected.

Also, a diffusion layer 44B formed of a porous substance is formed so as to completely cover the pair of the electrodes 42a, thereby enabling adjustment of the diffusion rate of the gas-to-be-measured which flows into the ammonia sensor portion 42 from the ambient atmosphere. The diffusion layer 44B prevents a short circuit between the paired electrodes 42a and enhances poisoning resistance and resistance to water adhesion.

In order to specify positional relations, which will be described below, between the ammonia sensor portion 42 and the inner gas introduction holes 143a and the inner gas discharge hole 143b of the protector 141, a region which encompasses the outlines of the ammonia sensor solid electrolyte body 25 and a pair of the electrodes 42a is considered to be the ammonia sensor portion 42 (the domain of the ammonia sensor portion 42). In the present embodiment, the porous diffusion layer 44B is formed on the surface of a pair of the electrodes 42a. Since the gas-to-be-measured stagnates in the diffusion layer 44B because of resistance to flow, a flow of the gas-to-be-measured in the diffusion layer 44B differs from a free flow of the gas-to-be-measured in a region outside the ammonia sensor portion 42. Thus, the region considered to be the ammonia sensor portion 42 (the domain of the ammonia sensor portion 42) encompasses the diffusion layer 44B.

For example, a region 42R ranging from the front end to the rear end of the diffusion layer 44B with respect to the axial direction O and ranging from the diffusion layer 44B to the ammonia sensor portion solid electrolyte body 25 with respect to the lamination direction perpendicular to the axial direction O is the domain of the ammonia sensor portion 42 (FIG. 2 shows the ranges of the region 42R with respect to the axial direction O and the direction perpendicular to the axial direction O, respectively).

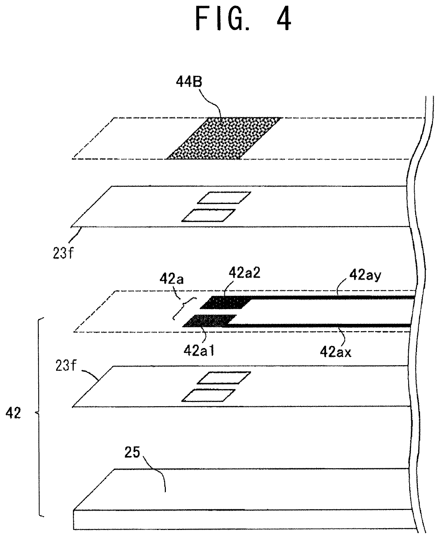

FIG. 4 is an exploded perspective view showing the configuration of the ammonia sensor portion 42. A pair consisting of electrodes 42a1 and 42a2 (which are collectively referred to as a pair of the electrodes 42a) is formed on the ammonia sensor portion solid electrolyte body 25. Leads 42ax and 42ay extend from the electrodes 42a1 and 42a2, respectively, along the longitudinal direction of the ammonia sensor portion solid electrolyte body 25. The leads 42ax and 42ay are covered with the insulation layer 23f from above and from underneath. However, right ends of the leads 42ax and 42ay are exposed without being covered with the insulation layer 23f and form predetermined electrode terminal portions (not shown), respectively.

The electrodes 42a1 and 42a2 are spaced apart from each other along the lateral direction of the ammonia sensor portion solid electrolyte body 25. The electrode 42a1 is formed of a material which predominantly contains gold and metal oxides, and functions as a detection electrode. The electrode 42a2 is formed of a material which predominantly contains platinum, and functions as a reference electrode. Since the detection electrode 42a1 is higher in reactivity with ammonia than the reference electrode 42a2, electromotive force is generated between the detection electrode 42a1 and the reference electrode 42a2.

Also, metal oxides contained in the electrode 42a1 function to burn flammable gas components other than ammonia in the gas-to-be-measured and to cause selective reaction of ammonia. Thus, ammonia in the gas-to-be-measured can be detected without being influenced by flammable gas components. Examples of the metal oxides include vanadium oxide (V₂O₅), bismuth oxide (Bi₂O₃), cobalt oxide (Co₃O₄) and germanium oxide (GeO₂). Particularly, bismuth vanadium oxide (BiVO₄) and cobalt oxide (Co₃O₄) are preferred metal oxides.

In view of adhesion to the ammonia sensor portion solid electrolyte body 25, the electrodes 42a1 and 42a2 may contain a ceramic material, such as zirconia or alumina.

Also, the ammonia sensor portion solid electrolyte body 25 is formed of an oxygen ion conductive material, such as ZrO₂. The leads 42ax and 42ay are formed of a material which predominantly contains, for example, platinum.

The diffusion layer 44B is formed of, for example, a material selected from the group consisting of alumina, spinel (MgAl₂O₄), silica alumina and mullite. By adjusting the thickness of the diffusion layer 44B and the particle size, particle size distribution, porosity, mixing ratio, etc., of the material, the gas diffusion time for reaching a selective reaction layer 42b and the electrodes 42a1 and 42a2 can be adjusted as desired.

Next, referring back to FIG. 2, the configuration of the controller 300 and a method of measuring NOₓ and ammonia concentrations will be described. The controller 300 is configured such that a microcomputer 60 and an analog control circuit 59 are mounted on a circuit board. The microcomputer 60 controls the controller 300 and includes a CPU (central processing unit) 61, a RAM 62, a ROM 63, a signal input/output section 64, an A/D converter 65, and an unillustrated clock. The CPU executes programs stored in the ROM 63, etc.

The control circuit 59 includes a reference voltage comparison circuit 51, an Ip1 drive circuit 52, a Vs detection circuit 53, an Icp supply circuit 54, an Ip2 detection circuit 55, a Vp2 application circuit 56, a heater drive circuit 57, and an ammonia sensor portion electromotive force detection circuit 58, which are described in detail below.

The control circuit 59 controls the NOₓ sensor portion 30, detects a first pumping current Ip1 and a second pumping current Ip2 which flow to the NOₓ sensor portion 30, and outputs the detected current data to the microcomputer 60.

The ammonia sensor portion electromotive force detection circuit 58 detects an ammonia concentration output (electromotive force) between the paired electrodes 42a1 and 42a2 and outputs the detected electromotive force data to the microcomputer 60.

More specifically, the outer first pumping electrode 2c of the NOₓ sensor portion 30 is connected to the IP1 drive circuit 52; the reference electrode 6c is connected to the Vs detection circuit 53 and the Icp supply circuit 54 in parallel; and the second pumping counter electrode 4c is connected to the Ip2 detection circuit 55 and the Vp2 application circuit 56 in parallel. The heater circuit 57 is connected to the heater 21.

A pair consisting of the electrodes 42a1 and 42a2 of the ammonia sensor portion 42 is connected to the ammonia sensor portion electromotive force detection circuit 58.

The circuits 51 to 56 have the following functions.

The Ip1 drive circuit 52 supplies the first pumping current Ip1 between the inner first pumping electrode 2b and the outer first pumping electrode 2c and detects the supplied first pumping current Ip1.

The Vs detection circuit 53 detects a voltage Vs between the detection electrode 6b and the reference electrode 6c and outputs the detected voltage Vs to the reference voltage comparison circuit 51.

The reference voltage comparison circuit 51 compares a reference voltage (e.g., 425 mV) and an output (voltage Vs) of the Vs detection circuit 53 and outputs the result of comparison to the Ip1 drive circuit 52. The Ip1 drive circuit 52 controls the direction and magnitude of the Ip1 current so that the voltage Vs becomes equal to the above-mentioned reference voltage, thereby adjusting oxygen concentration in the first measuring chamber S1 to a predetermined value set so as not to decompose NOₓ.

The Icp supply circuit 54 supplies a very weak current Icp to the detection electrode 6b and the reference electrode 6c so as to transport oxygen from the first measuring chamber S 1 to the reference oxygen chamber 15, thereby exposing the reference electrode 6c to a predetermined reference oxygen concentration.

The Vp2 application circuit 56 applies, between the inner second pumping electrode 4b and the second pumping counter electrode 4c, a fixed voltage Vp2 (e.g., 450 mV) set so as to decompose NOₓ gas contained in the gas-to-be-measured into oxygen and N₂ gas, thereby decomposing NOₓ into nitrogen and oxygen.

The Ip2 detection circuit 55 detects the second pumping current Ip2 which flows to the second pumping cell 4, at the time when oxygen generated through decomposition of NOₓ is pumped out from the second measuring chamber S2 toward the second pumping counter electrode 4c via the second solid electrolyte body 4a.

The Ip1 drive circuit 52 outputs a detected value of the first pumping current Ip1 to the A/D converter 65. Also, the Ip2 detection circuit 55 outputs a detected value of the second pumping current Ip2 to the A/D converter 65.

The A/D converter 65 digitizes these values and outputs the digitized values to the CPU 61 via the signal input/output section 64.

Next, an example of control using the control circuit 59 is described. First, upon receipt of power from an external power supply in association with start of the engine, the heater circuit 57 activates the heater 21, thereby heating the first pumping cell 2, the oxygen concentration detection cell 6, and the second pumping cell 4 to an activation temperature. Also, the Icp supply circuit 54 supplies a very weak current Icp which flows between the detection electrode 6b and the reference electrode 6c so as to transport oxygen from the first measuring chamber S1 into the reference oxygen chamber 15, thereby establishing oxygen reference.

When the NOₓ sensor portion 30 is heated to an appropriate temperature by means of the heater 21, the ammonia sensor portion 42 on the NOₓ sensor portion 30 is also heated to a desired temperature.

When the cells are heated to the activation temperature, the first pumping cell 2 pumps out oxygen contained in the gas-to-be-measured (exhaust gas) which has flowed into the first measuring chamber S1, from the inner first pumping electrode 2b toward the outer first pumping electrode 2c.

At this time, since oxygen concentration in the first measuring chamber S1 assumes a value corresponding to the electrode-to-electrode voltage (terminal-to-terminal voltage) Vs of the oxygen concentration detection cell 6, the Ip1 drive circuit 52 controls the first pumping current Ip1 which is supplied to the first pumping cell 2, in order for the electrode-to-electrode voltage Vs to become the above-mentioned reference voltage. In this manner, the oxygen concentration in the first measuring chamber S1 is adjusted to a level at which NOₓ does not decompose.

The gas-to-be-measured whose oxygen concentration has been adjusted flows toward the second measuring chamber S2. The Vp2 application circuit 56 applies, between the electrodes (terminals) of the second pumping cell 4, a fixed voltage Vp2 (e.g., 450 mV) set so as to decompose NOₓ gas contained in the gas-to-be-measured into oxygen and N₂ gas, thereby decomposing NOₓ into nitrogen and oxygen. The second pumping current Ip2 is supplied to the second pumping cell 4 so as to pump out oxygen generated by decomposition of NOₓ from the second measuring chamber S2. At this time, since the second pumping current Ip2 and NOₓ concentration are in linear relation with each other, NOₓ concentration in the gas-to-be-measured can be detected by means of the Ip2 detection circuit 55 detecting the second pumping current Ip2.

Also, ammonia concentration in the gas-to-be-measured can be detected by means of the ammonia sensor portion electromotive force detection circuit 58 detecting ammonia concentration output (electromotive force) between the paired electrodes 42a1 and 42a2. Ammonia concentration equivalent values determined on the basis of the electromotive force generated between the electrodes 42a1 and 42a2 (the rate of change between a base value of electromotive force as measured when ammonia concentration is 0 and a value of electromotive force as measured when ammonia is present (sensitivity) can also be used) are stored beforehand in the microcomputer 60. On the basis of the stored values, NH₃ concentration is calculated.

The multigas sensor 200A of the present invention can be applied to, for example, detection of deterioration in catalyst provided as an accessory of an engine system, optimization of the amount of injection of urea in a urea SCR system, and accurate measurement of post-catalyst gas components (NOₓ and ammonia). For example, in the case of using only an NOₓ sensor, a sensing system apparatus cannot clearly differentiate which one of the following is a cause of post-catalytic discharge of ammonia: (i) discharge of ammonia due to excessive addition of urea; (ii) discharge of NOₓ due to excessively small addition of urea; and (iii) discharge of ammonia associated with deterioration in SCR catalyst. In contrast, use of the multigas sensor of the present invention can differentiate which one of the above is causing post-catalytic discharge of ammonia.

The multigas sensor 200A of the present invention can be manufactured in a manner similar to that employed in manufacture of publicly known NOₓ sensors and ammonia sensors. For example, similar to manufacture of a publicly known NOₓ sensor, the solid electrolyte bodies of the NOₓ sensor portion are formed from a green sheet. The electrodes, the leads, and the insulation layers are paste-printed on the green solid electrolyte bodies, thereby forming a green body of the NOₓ sensor portion. Next, a green body of the ammonia sensor portion is formed at a predetermined position on the surface of the green body of the NOₓ sensor portion. The green body of the ammonia sensor portion can be formed by paste-printing, on the green body of the NOₓ sensor portion, the electrodes, the leads, the sensitive section, the solid electrolyte body, the diffusion layer, etc., which constitute the ammonia sensor portion.

The green body of the NOₓ sensor portion on which the green body of the ammonia sensor portion is formed is fired at a predetermined temperature, to thereby manufacture the gas sensor element of the multigas sensor. The gas sensor element is assembled to the housing, thereby yielding the multigas sensor. As for the electrode 42a1 and the protection layer 44, subsequent to firing of other components, the electrode 42a1 and the protection layer 44 may be subjected to heat treatment at a temperature lower than the firing temperature.

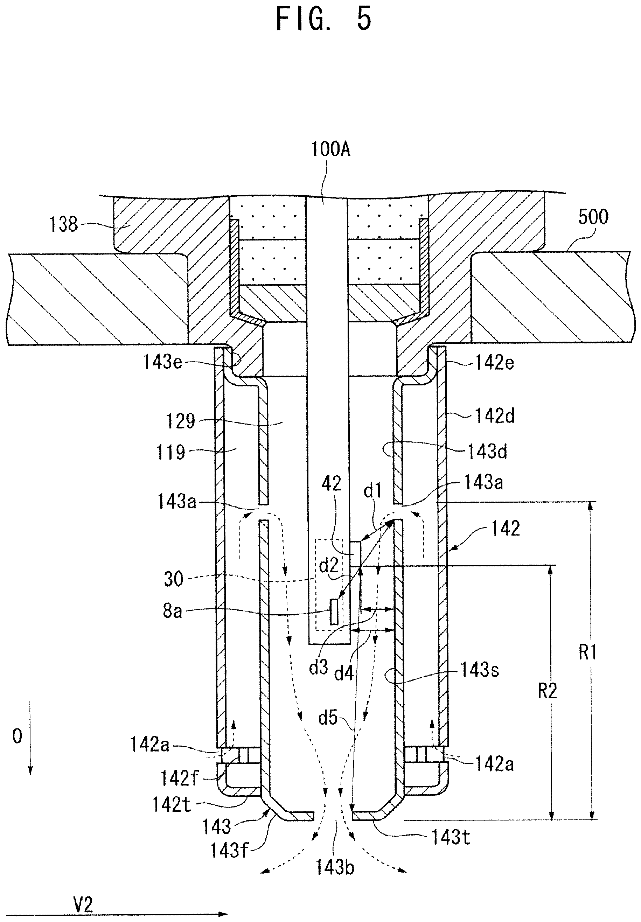

Next, referring to FIG. 5, the positional relations of the inner gas introduction holes 143a and the inner gas discharge hole 143b of the protector 141 with the NOₓ sensor portion 30 and the ammonia sensor portion 42 will be described.

First, the protector 141 is described in detail. The protector 141 has a dual structure composed of the inner protector 143 and the tubular outer protector 142. The inner protector 143 has an inner side wall 143d and the front end wall 143t located at the front end of the inner side wall 143d. An outer side wall 142d of the tubular outer protector 142 radially surrounds the inner side wall 143d of the inner protector 143. A gap is formed between the inner side wall 143d of the inner protector 143 and the outer side wall 142d of the outer protector 142, thereby forming a gas chamber 119.

A rear proximal end portion 143e of the inner protector 143 is expanded in diameter so as to engage the outer circumference of a front end portion of the metallic shell 138. A peripheral portion of the front end wall 143t is formed into a taper portion 143f which expands in a tapered condition toward the inner side wall 143d. The inner side wall 143d has a plurality of (in the present embodiment, six) the inner gas introduction holes 143a disposed along the circumferential direction at positions located toward the rear end thereof. The inner gas introduction holes 143a are adapted to introduce the gas-to-be-measured which has been introduced into the gas chamber 119 through the outer gas introduction holes 142a of the outer protector 142, which will be described below, into the interior of the inner protector 143; i.e., into a gas detection chamber 129 where the front end portion 105 of the gas sensor element 100A is disposed.

Furthermore, the front end wall 143t of the inner protector 143 has the inner gas discharge hole 143b. The inner gas discharge hole 143b is adapted to discharge, to the ambient atmosphere, gas which is introduced into the gas detection chamber 129 through the inner gas introduction holes 143a.

A rear proximal end portion 142e of the outer protector 142 is engaged with the outer circumference of the proximal end portion 143e of the inner protector 143. Specifically, while the proximal end portion 142e of the outer protector 142 is externally fitted to the proximal end portion 143e of the inner protector 143, they are full-circle laser-welded together.

The bottom wall 142t of the outer protector 143 is bent radially inward in the vicinity of the taper portion 143f of the inner protector 143, thereby closing the gas chamber 119 at its front end.

Furthermore, the outer side wall 142d of the outer protector 142 has a plurality of (in the present embodiment, six) the outer gas introduction holes 142a formed along the circumferential direction, for establishing communication between the ambient atmosphere of the outer protector 142 and the gas chamber 119. The outer gas introduction holes 142a are located frontward of the inner gas introduction holes 142a of the inner protector 142 with respect to the axial direction O. Louvers 142f extending radially inward (toward the gas chamber 119) are provided in the outer gas introduction holes 142a. The louvers 142f impart a swirl around the inner side wall 143d of the inner protector 143 to exhaust gas which is introduced into the gas chamber 119 from the ambient atmosphere through the outer gas introduction holes 142a.

According to the present invention, in the case of a protector having a multi-wall structure, the gas introduction holes 143a of the innermost protector (the inner protector 143) correspond to "a gas introduction hole" in the present invention. Similarly, an inner surface 143s of the inner side wall 143d of the innermost protector (the inner protector 143) corresponds to "an inner surface of the side wall of the protector" appearing in claims.

The multigas sensor 200A is fixed to an exhaust pipe 500 by means of the threaded portion 139, and the inner gas discharge hole 143b is located toward the center of the exhaust pipe 500 with respect to the inner gas introduction holes 143a. The gas-to-be-measured V₂ which flows through the exhaust pipe 500 changes its direction of flow by the effect of the taper portion 143f of the inner protector 143, thereby generating negative pressure in the vicinity of the front end wall 143t of the inner protector 143. By virtue of this phenomenon, the gas-to-be-measured flows within the protector 141 from the outer gas introduction holes 142a to the inner gas discharge hole 143b by way of the inner gas introduction holes 143a (flows indicated by the broken lines of FIG. 5).

Thus, when a shortest distance d₁ between the inner gas introduction hole 143a and the ammonia sensor portion 42 is rendered shorter than a shortest distance d₂ between the inner gas introduction hole 143a and the gas diffusion hole 8a, the gas-to-be-measured which has passed through the gas chamber 119 first comes in contact with the ammonia sensor portion 42 close to the inner gas introduction hole 143a. Accordingly, the gas-to-be-measured which has been introduced into the gas detection chamber 129 through the inner gas introduction holes 143a does not pass in the vicinity of the NOₓ sensor portion 30, whose temperature is raised, before reaching the ammonia sensor portion 42. Therefore, the fresh gas-to-be-measured before undergoing thermal decomposition of ammonia can reach the ammonia sensor portion 42, thereby improving accuracy in detecting ammonia concentration. Also, since the temperature of the NOₓ sensor portion 30 can be controlled to be sufficiently high, accuracy in measuring NOₓ concentration also improves.

Notably, the shortest distances d₁ and d₂ are shortest distances in a three-dimensional space. For example, in the case where a plurality of the gas introduction holes 143a and a plurality of the gas diffusion holes 8a are provided, the shortest one of associated distances is employed.

According to the present invention, at least a subportion of the ammonia sensor portion 42 is disposed within a positional range R₁ along the axial direction O between the inner gas introduction hole 143a and the inner gas discharge hole 143b. Thus, the ammonia sensor portion 42 is present on a gas-to-be-measured flow path (a flow indicated by the arrows of FIG. 5) from the inner gas introduction hole 143a to the inner gas discharge hole 143b. Therefore, the ammonia sensor portion 42 can measure ammonia concentration in such a condition that sufficient gas-to-be-measured is supplied (diffused) thereto. Accordingly, even when the flow rate of the gas-to-be-measured varies, the ammonia sensor portion 42 can stably measure ammonia concentration. As a result, the ammonia sensor portion 42 exhibits high responsiveness in ammonia concentration detection. By contrast, in the case where the ammonia sensor portion 42 is not disposed within the positional range R₁, the ammonia sensor portion 42 is not present on the gas-to-be-measured flow path from the inner gas introduction hole 143a to the inner gas discharge hole 143b. Thus, for example, the gas-to-be-measured whose flow is unstabilized due to turbulence or the like reaches the ammonia sensor portion 42. Accordingly, sufficient gas-to-be-measured is not supplied (diffused) to the ammonia sensor portion 42. As a result, upon variation in the flow rate of the gas-to-be-measured, the ammonia concentration output becomes unstable, and responsiveness in ammonia concentration detection deteriorates.

The expression "at least a subportion of the ammonia sensor portion 42 is disposed within a positional range R₁" means that the following condition suffices: a subportion of the ammonia sensor portion 42 is disposed within a region corresponding to the positional range R₁. For example, in the case where, as viewed along the axial direction O, the ammonia sensor portion 42 is located most rearward relative to the inner gas introduction hole 143a, the front end of the ammonia sensor portion 42 coincides with the rear end of the inner gas introduction hole 143a. The domain of the ammonia sensor portion 42 (e.g., the region 42R shown in FIG. 2) is as mentioned above.

In the present embodiment, the gas diffusion holes 8a are disposed within a positional range R₂ along the axial direction O between the inner gas discharge hole 143b and the ammonia sensor portion 42. By employing this arrangement, the gas diffusion holes 8a are present on gas-to-be-measured flow paths (flows indicated by the arrows of FIG. 5) from the inner gas introduction holes 143a to the inner gas discharge hole 143b. Therefore, the NOₓ sensor portion 30 can measure NOₓ concentration in such a condition that sufficient gas-to-be-measured is supplied (diffused) thereto. Thus, responsiveness in NOₓ detection improves; as a result, the NOₓ sensor portion 30 can stably measure NOₓ concentration.

The "positional range R₂ between the inner gas discharge hole 143b and the ammonia sensor portion 42" means a positional range along the axial direction O which does not encompass the ammonia sensor portion 42; i.e., a region located frontward of the front end of the ammonia sensor portion 42.

Furthermore, according to the present embodiment, the ammonia sensor portion 42 projects from the gas sensor element 100A. Thus, a shortest distance d₃ between the ammonia sensor portion 42 and the inner surface 143s of the inner side wall 143d of the inner protector 143 is shorter than a shortest distance d₄ between the gas sensor element 100A excluding the ammonia sensor portion 42 and the inner surface 143s of the inner side wall 143d of the inner protector 143.

By employing the above configuration, by means of the venturi effect, the flow rate of the gas-to-be-measured in a gap (the gas detection chamber 129) between the inner protector 143 and the gas sensor element 100A as measured in the vicinity of the ammonia sensor portion 42 is higher than the flow rate of the gas-to-be-measured in the gap as measured in the other region. Thus, sufficient gas-to-be-measured is supplied (diffused) to the ammonia sensor portion 42, thereby further improving accuracy in measuring ammonia concentration.

Furthermore, according to the present embodiment, the NOₓ sensor portion 30 and the ammonia sensor portion 42 overlap each other with respect to the axial direction O. Thus, in contrast to the case where the NOₓ sensor portion 30 and the ammonia sensor portion 42 do not overlap each other (the sensor portions 30 and 42 are located away from each other) with respect to the axial direction O, the sensor portions 30 and 42 can be exposed to substantially the same gas-to-be-measured. Thus, NOₓ concentration and ammonia concentration can be measured with higher accuracy.

Also, according to the present embodiment, the shortest distance d₁ between the ammonia sensor portion 42 and the inner gas introduction hole 143a is shorter than a shortest distance d₅ between the ammonia sensor portion 42 and the inner gas discharge hole 143b. Thus, the gas-to-be-measured in a condition before diffusion toward the inner gas discharge hole 143b and similar to the condition of the gas-to-be-measured which passes through the inner gas introduction hole 143a can reach the ammonia sensor portion 42. Thus, accuracy in measuring ammonia concentration improves.

The present invention is not limited to the above embodiment, but the idea and the scope of the present invention cover various modifications and equivalents. For example, in the above-described embodiment, the NOₓ sensor portion 30 includes three layers of the solid electrolyte body. However, two layers of the solid electrolyte body may be provided. The structure of an NOₓ sensor portion having two layers of the solid electrolyte body is described in, for example, Japanese Patent Application Laid-Open (*kokai*) No. 2004-354400 (FIG. 2).

In this case, the second measuring chamber S2 is formed between the solid electrolyte bodies 2a and 6a in FIG. 2, and the second diffusion resistor 8b separates the first measuring chamber S1 and the second measuring chamber S2 from each other. The inner second pumping electrode 4b is disposed on the upper surface of the solid electrolyte body 6. The lower surface of the solid electrolyte body 6 is exposed to the ambient atmosphere, and the second pumping counter electrode 4c is disposed on the exposed surface.

Also, the above-described embodiment uses the protector 141 having a dual structure composed of the inner protector 143 and the outer protector 142. However, the present invention is not limited thereto. The protector may have a mono-structure or a triplex or higher structure.

Also, the above embodiment is described while mentioning the gas sensor element 100A having the insulation layer 23f and the ammonia sensor portion solid electrolyte body 25 provided in such a manner as to extend in the longitudinal direction of the gas sensor element 100A. However, the present invention is not limited thereto. The insulation layer 23f and the ammonia sensor portion solid electrolyte body 25 may be provided merely in the vicinity of the ammonia sensor portion 42.

Also, in the above-described embodiment, the electrode 42a1 of the ammonia sensor portion 42 is formed of a material which predominantly contains gold and metal oxides. However, the present invention is not limited thereto. The electrode 42a1 may be formed of a material which predominantly contains gold, while being covered with a selective reaction layer which predominantly contains metal oxides.

Also, in the above-described embodiment, NOₓ concentration and ammonia concentration are detected on the basis of NOₓ concentration output and ammonia concentration output, respectively. However, the present invention is not limited thereto. One of NOₓ concentration output and ammonia concentration output may be used as a correction value for the other.

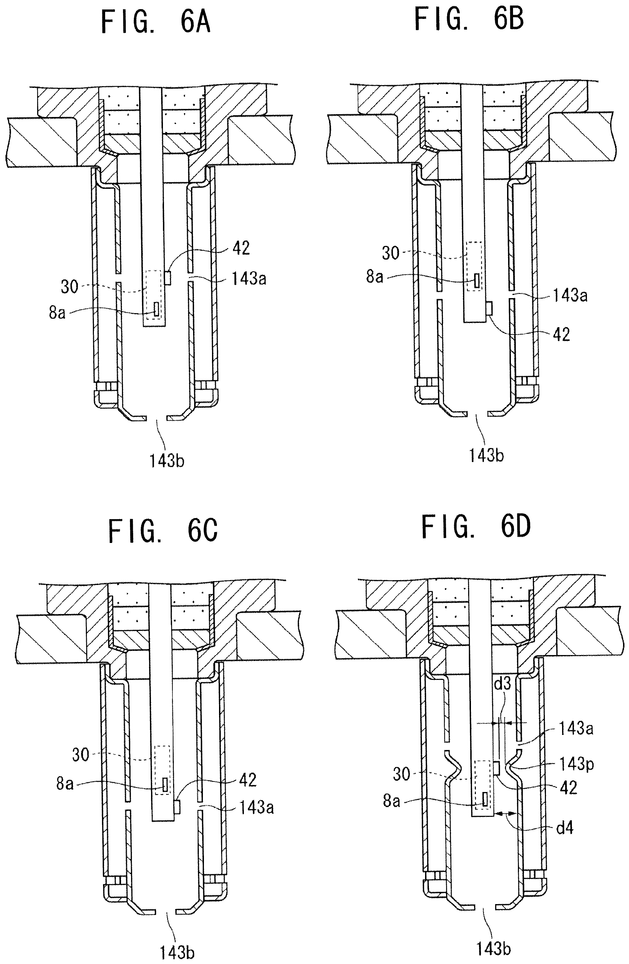

Furthermore, in the above-described embodiment, the gas diffusion holes 8a are disposed within the positional range R₂ along the axial direction between the inner gas discharge hole 143b and the ammonia sensor portion 42. However, the present invention is not limited thereto. As shown in FIGS. 6B and 6C, the gas diffusion holes 8a may be disposed rearward of the inner gas introduction holes 143a.

Furthermore, in the above-described embodiment, the NOₓ sensor portion 30 and the ammonia sensor portion 42 overlap each other with respect to the axial direction O. However, the present invention is not limited thereto. As shown in FIGS. 6B and 6C, the NOₓ sensor portion 30 and the ammonia sensor portion 42 may not overlap each other.

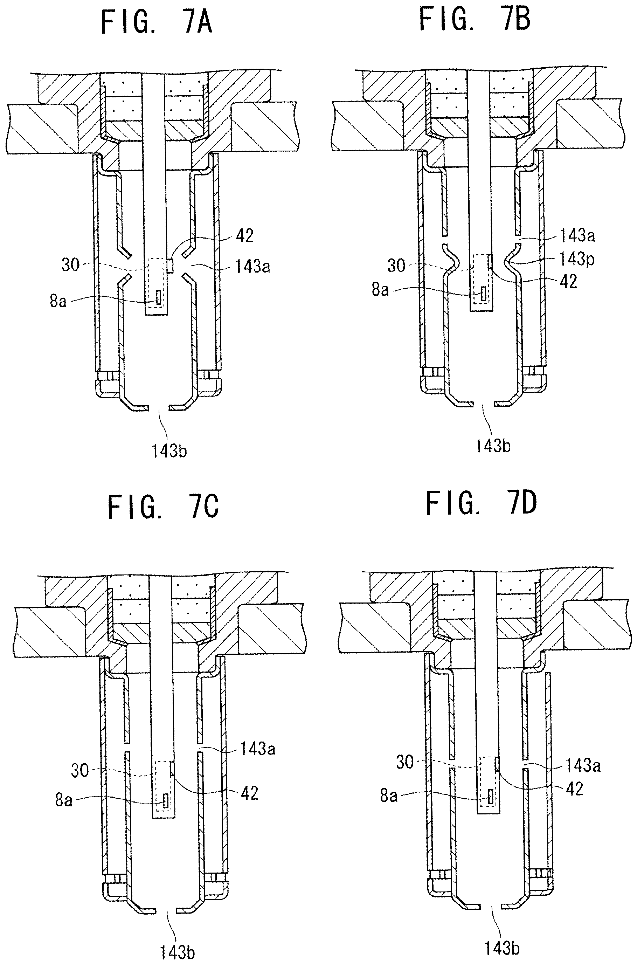

Furthermore, in the above-described embodiment, the ammonia sensor portion 42 projects from the outer surface of the gas sensor element 100A so as to render the shortest distance d₃ shorter than the shortest distance d₄. However, the present invention is not limited thereto. As shown in FIGS. 6D, 7A, and 7B, a portion of the inner side wall 143d of the inner protector 143 whose distance to the ammonia sensor portion 42 is the shortest distance between the ammonia sensor portion 42 and the inner side wall 143d may project radially inward, thereby forming a diameter-reduced portion 143p. In association with formation of the diameter-reduced portion 143p of the inner protector 143, a corresponding portion of the outer side wall 142d of the outer protector 142 may be reduced in diameter.

Furthermore, in the above-described embodiment, the ammonia sensor portion 42 projects from the outer surface of the gas sensor element 100A. However, the present invention is not limited thereto. As shown in FIGS. 7B, 7C, and 7D, the ammonia sensor portion 42 may be embedded in the front end portion 105 of the gas sensor element 100A while exposing at the outer surface of the front end portion 105.

### EXAMPLES

### (1) Fabrication of sensors

The multigas sensor 200A according to the above-described embodiment (FIGS. 1 to 5) was fabricated. The inner first pumping electrode 2b, the outer first pumping electrode 2c, the detection electrode 6b, the reference electrode 6c, the inner second pumping electrode 4b, and the outer second pumping electrode 4c of the NOₓ sensor portion 30 predominantly contained platinum. The electromotive-force-type ammonia sensor portion 42 employed a pair of the electrodes 42a consisting of the detection electrode 42a1 which contained 10% by mass Ca₃O₄, 5% YSZ, and a balance of gold, and the reference electrode 42a2 which predominantly contained platinum. A porous layer of alumina (Al₂O₃) was formed as the diffusion layer 44B which covers a pair of the electrodes 42a. The multigas sensor 200A shown in FIGS. 1 to 5 is referred to as "Example 1."

Similarly, multigas sensors of Examples 2 to 9 and Comparative Examples 1 to 3 as shown in FIGS. 6 to 8 were fabricated while the positions of the NOₓ sensor portion 30 and the ammonia sensor portion 42 of the gas sensor element 100A and the positions of the inner gas introduction holes 143a and the inner gas discharge hole 143b were varied.

Example 2 is a modification of Example 1 in which, as shown in FIG. 6A, the inner gas introduction holes 143a are moved to a position corresponding to the center of the ammonia sensor portion 42 with respect to the axial direction O.

Example 3 is a modification of Example 1 in which, as shown in FIG. 6B, the NOₓ sensor portion 30 is moved to a position located rearward of the inner gas introduction holes 143a with respect to the axial direction O.

Example 4 is a modification of Example 3 in which, as shown in FIG. 6C, the inner gas introduction holes 143a are moved to a position corresponding to the center of the ammonia sensor portion 42 with respect to the axial direction O.

Example 5 is a modification of Example 1 in which, as shown in FIG. 6D, a portion of the inner side wall 143d of the inner protector 143 which faces the ammonia sensor portion 42 is reduced in diameter, thereby forming the diameter-reduced portion 143p.

Example 6 is a modification of Example 2 in which, as shown in FIG. 7A, the peripheries of the inner gas introduction holes 143a are reduced in diameter.

Example 7 is a modification of Example 5 in which, as shown in FIG. 7B, the outermost surface of the ammonia sensor portion 42 is flush with the outer surface of the gas sensor element 100A (i.e., the ammonia sensor portion 42 is embedded in the front end portion 105 of the gas sensor element 100A).

Example 8 is a modification of Example 1 in which, as shown in FIG. 7C, the outermost surface of the ammonia sensor portion 42 is flush with the outer surface of the gas sensor element 100A (i.e., the ammonia sensor portion 42 is embedded in the front end portion 105 of the gas sensor element 100A).

Example 9 is a modification of Example 2 in which, as shown in FIG. 7D, the outermost surface of the ammonia sensor portion 42 is flush with the outer surface of the gas sensor element 100A (i.e., the ammonia sensor portion 42 is embedded in the front end portion 105 of the gas sensor element 100A).

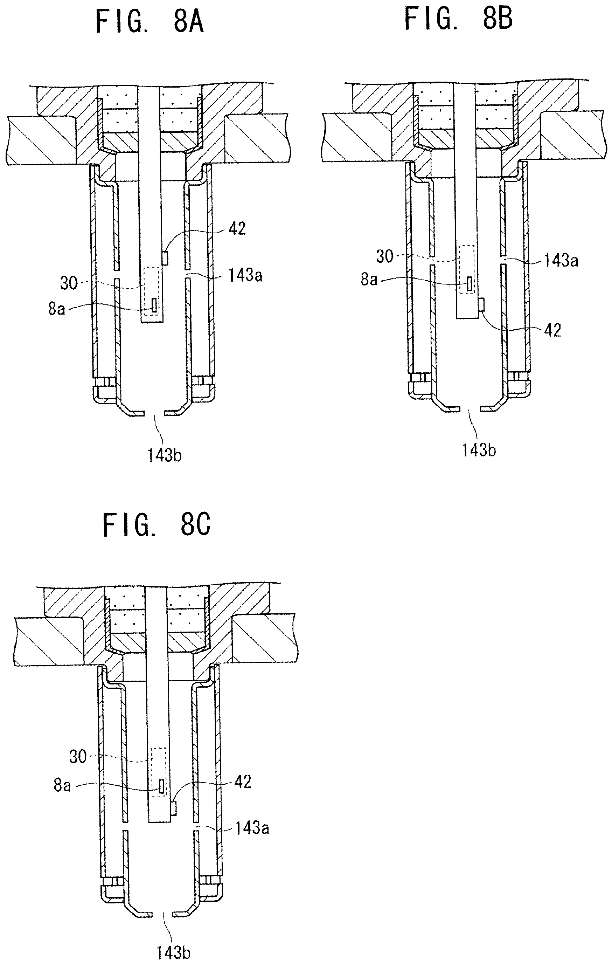

Meanwhile, Comparative Example 1 is a modification of Example 2 in which, as shown in FIG. 8A, the position of the ammonia sensor portion 42 is moved rearward of the gas introduction holes 143a.

Comparative Example 2 is a modification of Example 3 in which, as shown in FIG. 8B, the position of the gas introduction holes 143a along the axial direction O is moved rearward of the gas diffusion holes 8a.

Comparative Example 3 is a modification of Example 3 in which, as shown in FIG. 8C, the position of the gas introduction holes 143a along the axial direction O is moved frontward of the ammonia sensor portion 42.

Table 1 shows the positional relations of the inner gas introduction holes 143a and the inner gas discharge hole 143b of the inner protector 143 with the NOₓ sensor portion 30 and the ammonia sensor portion 42 in the Examples and the Comparative Examples.

### (2) Evaluation of sensor characteristics

### 2-1 Accuracy in detection of ammonia concentration

By use of a model gas generator, sensor characteristics were evaluated. The gas composition of the model gas generator was changed by changing NH₃ content to 20 ppm and 50 ppm while other gas contents were as follows: O₂: 10%; CO₂: 5%; H₂O: 5%; N₂: balance. Other test conditions were as follows: gas temperature: 280°C; gas flow rate: 7.5 m/s; controlled temperature in a central region between the paired electrodes 42a of the ammonia sensor portion 42: 600°C.

The multigas sensors were disposed in a gas flow of the model gas generator. NH₃ was added to the gas flow in an amount of 20 ppm and 50 ppm, and the ammonia concentration output (EMF) of the ammonia sensor portion 42 was detected.

### 2-2 Flow rate dependency of ammonia concentration detection

The gas composition of the model gas generator was varied by changing NH₃ content in a range of 0 ppm to 50 ppm while other gas contents were as follows: O₂: 10%; CO₂: 5%; H₂O: 5%; N₂: balance. Also, the gas flow rate was changed to 1.2 m/s, 3.7 m/s, and 7.5 m/s while other test conditions were as follows: gas temperature: 280°C; controlled temperature in a central region between the paired electrodes 42a of the ammonia sensor portion 42: 600°C.

The multigas sensors were disposed in a gas flow of the model gas generator. NH₃ was added in an amount of 0 ppm to 50 ppm to the gas flow whose flow rate was changed at mentioned above, and the ammonia concentration output (EMF) of the ammonia sensor portion 42 was detected.

### 2-3 Responsiveness in ammonia detection and NOₓ detection

The gas composition of the model gas generator was varied by changing the NH₃ (or NOₓ) content from 0 ppm to 50 ppm or from 50 ppm to 0 ppm while other gas contents were as follows: O₂: 10%; CO₂: 5%; H₂O: 5%; N₂: balance. The gas temperature was 280°C, and the gas flow rate was 7.5 m/s. The controlled temperature in a central region between the paired electrodes 42a of the ammonia sensor portion 42 was 600°C; the controlled temperature of the first pumping cell 2 of the NOₓ sensor portion 30 was 750°C; and the controlled temperature of the second pumping cell 4 of the NOₓ sensor portion 30 was 600°C.

The multigas sensors were disposed in a gas flow of the model gas generator. During detection of sensor output in the gas flow whose NH₃ (or NOₓ) content was 0 ppm, the gas flow was changed to a gas flow which was supplied through a separate flow path and contained NH₃ (or NOₓ) in an amount of 50 ppm, by opening a solenoid valve. At this time, a change with time in the output of the ammonia sensor portion 42 (or the NOₓ sensor portion 30) was detected. Similarly, during detection of the output of the ammonia sensor portion 42 (or the NOₓ sensor portion 30) in the gas flow which was supplied through the separate flow path by opening the solenoid valve and contained NH₃ (or NOₓ) in an amount of 50 ppm, the gas flow was changed to a gas flow whose NH₃ (or NOₓ) content was 0 ppm by closing the solenoid valve. At this time, a change with time in the output of the ammonia sensor portion 42 (or the NOₓ sensor portion 30) was detected. Notably, a time of 0.02 sec required for changeover between the gases in the model gas generator is excluded from the results of detection of the sensor output.

In the experiment in which NH₃ (or NOₓ) content was sharply changed from 0 ppm to 50 ppm with the point of change of signal of the solenoid valve taken as time 0, the time which elapsed until the sensor output reached 63% of the saturated value of the sensor output was taken as the response time. Also, in the experiment in which NH₃ (or NOₓ) content was sharply changed from 50 ppm to 0 ppm, the time which elapsed until the sensor output was reduced to 63% of the saturated value of the sensor output was taken as the response time.

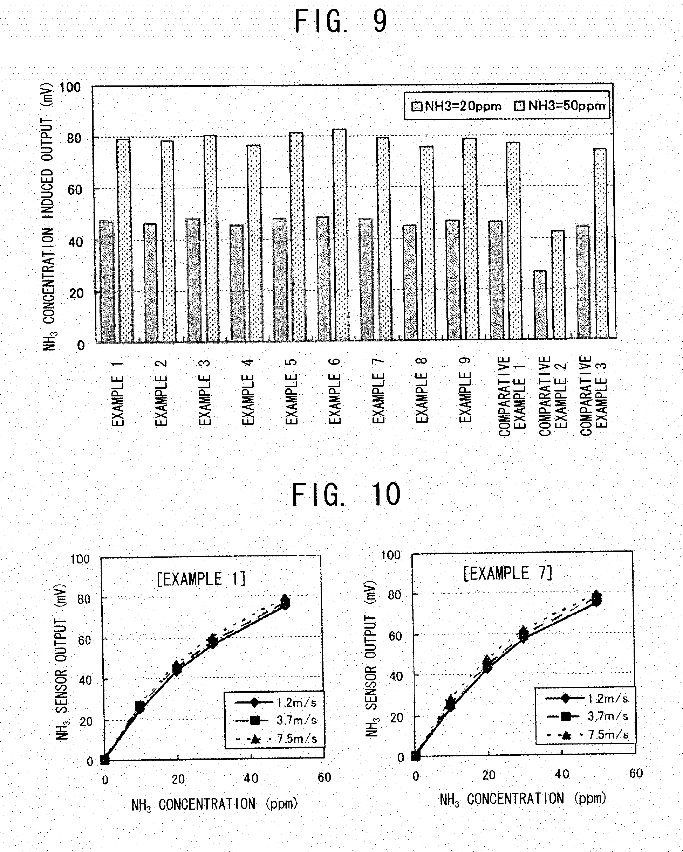

Table 1 and FIGS. 9 to 13 show the results of the experiments mentioned above. Evaluation of the detection characteristics of the ammonia sensor portion 42 and the NOₓ sensor portion 30 appearing in Table 1 is made under the following criteria: "Good": the rate of deviation from measured values of Example 1 is less than ±10%; "Fair": the rate of deviation is less than ±20%; and "Poor": the rate of deviation is ±20% or greater. The sensor portions evaluated as "Good" or "Fair" raise no problem in actual use.

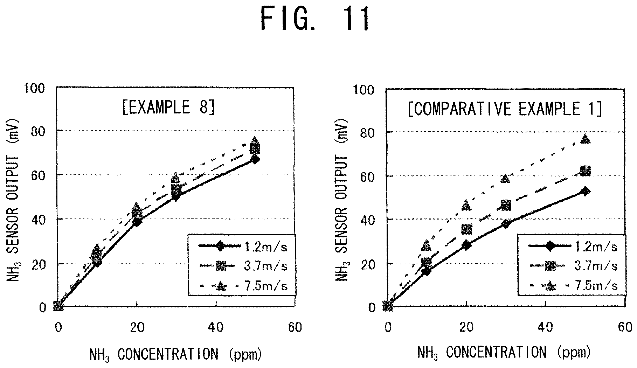

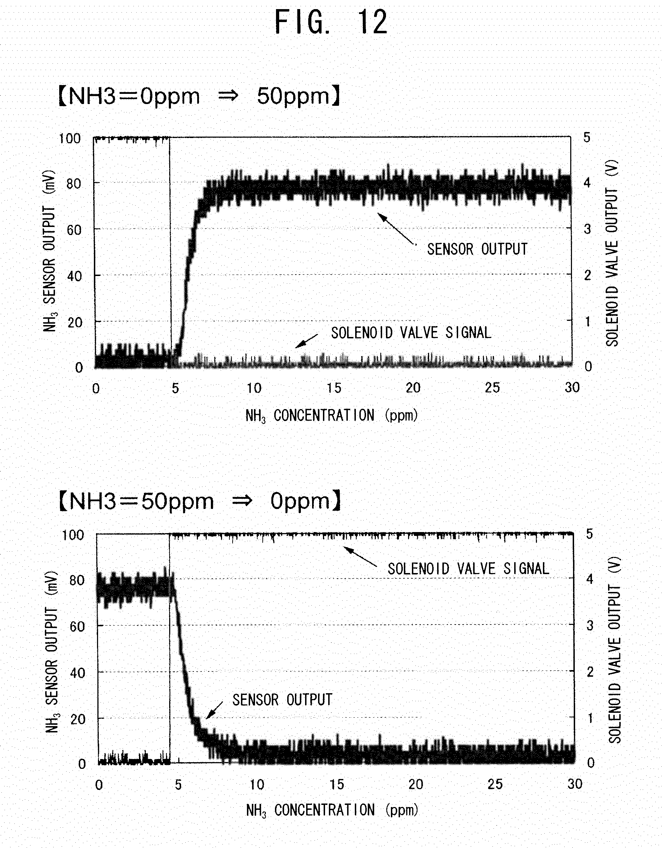

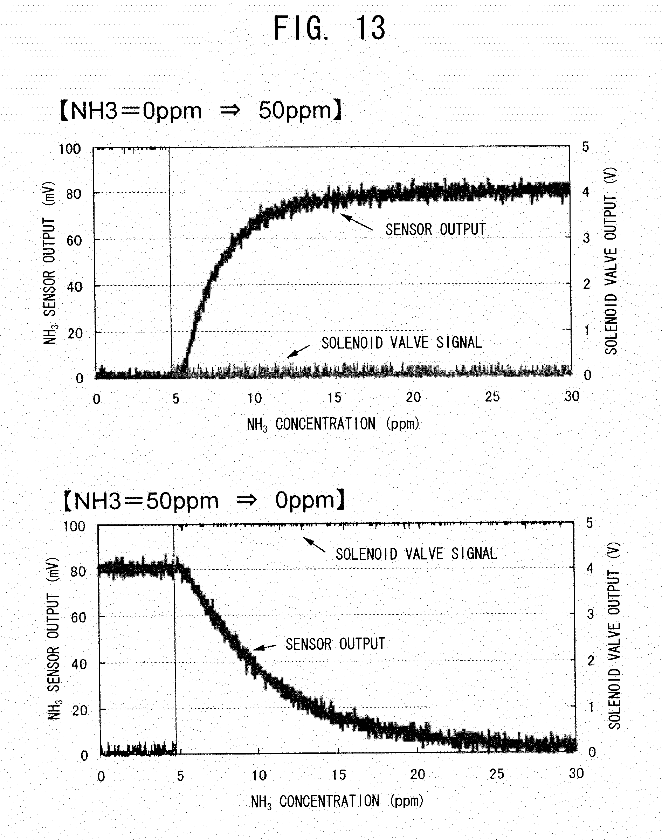

FIG. 9 shows accuracy in detection of ammonia concentration (ammonia concentration output at an NH₃ content of 20 ppm and 50 ppm) of the Examples and Comparative Examples. FIG. 10 shows the flow rate dependency of ammonia concentration detection of Examples 1 and 7. FIG. 11 shows the flow rate dependency of ammonia concentration detection of Example 8 and Comparative Example 1. FIG. 12 shows responsiveness in ammonia detection of Example 1. FIG. 13 shows the responsiveness in ammonia detection of Comparative Example 1.

**Table 1**

| | Axial position of ammonia sensor portion | Magnitude relation between d₁ and d₂ | Axial position of gas diffusion holes | Magnitude relation between d₃ and d₄ | Axial positions of NOₓ sensor portion and ammonia sensor portion | NH₃ detection characteristics of ammonia sensor portion | | | Responsiveness in NOₓ detection of NOₓ sensor portion |
|---|---|---|---|---|---|---|---|---|---|
| | | | | | | Accuracy of NH₃ detection | Gas flow rate dependency | Responsiveness | |
| Example 1 | Within positional range R₁ | d₁ < d₂ | Within positional range R₂ | d₃ < d₄ | Overlapping | Good | Good | Good | Good |
| Example 2 | Within positional range R₁ | d₁ < d₂ | Within positional range R₂ | d₃ < d₄ | Overlapping | Good | Good | Good | Good |
| Example 3 | Within positional range R₁ | d₁ < d₂ | Outside positional range R₂ | d₃ < d₄ | Nonoverlapping | Good | Good | Good | Fair |
| Example 4 | Within positional range R₁ | d₁ < d₂ | Outside positional range R₂ | d₃ < d₄ | Nonoverlapping | Good | Good | Good | Fair |
| Example 5 | Within positional range R₁ | d₁ < d₂ | Within positional range R₂ | d₃ < d₄ | Overlapping | Good | Good | Good | Good |
| Example 6 | Within positional range R₁ | d₁ < d₂ | Within positional range R₂ | d₃ < d₄ | Overlapping | Good | Good | Good | Good |
| Example 7 | Within positional range R₁ | d₁ < d₂ | Within positional range R₂ | d₃ < d₄ | Overlapping | Good | Good | Good | Good |
| Example 8 | Within positional range R₁ | d₁ < d₂ | Within positional range R₂ | d₃ = d₄ | Overlapping | Good | Fair | Fair | Good |
| Example 9 | Within positional range R₁ | d₁ < d₂ | Within positional range R₂ | d₃ = d₄ | Overlapping | Good | Fair | Fair | Good |
| Comp. Example 1 | Outside positional range R₁ | d₁ < d₂ | Within positional range R₂ | d₃ < d₄ | Nonoverlapping | Good | Poor | Poor | - |
| Comp. Example 2 | Within positional range R₁ | d₁ > d₂ | Outside positional range R₂ | d₃ < d₄ | Nonoverlapping | Poor | Good | Good | - |
| Comp. Example 3 | Outside positional range R₁ | d₁ < d₂ | Outside positional range R₂ | d₃ < d₄ | Nonoverlapping | Good | Poor | Poor | - |

As is apparent from Table 1 and FIGS. 9 to 13, in the case of Examples 1 to 9 in which at least a subportion of the ammonia sensor portion 42 is disposed within the positional range R₁, and d₁ is smaller than d₂, the gas-to-be-measured first comes in contact with the ammonia sensor portion 42 located close to the inner gas introduction holes 143a. Thus, the fresh gas-to-be-measured before coming into contact with the NOₓ sensor portion 30 can reach the ammonia sensor portion 42, thereby improving accuracy in detection of ammonia concentration.

By contrast, in the case of Comparative Examples 1 and 3 in which the ammonia sensor portion 42 is disposed outside the positional range R₁, the flow rate dependency of ammonia concentration detection increases, resulting in a great deterioration in responsiveness in ammonia detection. Conceivably, this is for the following reason. Since the ammonia sensor portion 42 is not present on gas-to-be-measured flow paths extending from the inner gas introduction holes 143a to the inner gas discharge holes 143b, for example, turbulence or the like causes a failure to supply (diffuse) sufficient gas-to-be-measured to the ammonia sensor portion 42. Thus, when the flow rate of the gas-to-be-measured varies, measured values of ammonia concentration fail to settle, resulting in deterioration in responsiveness in ammonia detection.

In the case of Comparative Example 2 in which d₁ is greater than d₂, accuracy in detection of ammonia concentration deteriorates greatly. Conceivably, this is for the following reason. Since the gas-to-be-measured passes in the vicinity of the NOₓ sensor portion 30 of high temperature before reaching the ammonia sensor portion 42, ammonia is thermally decomposed. Consequently, the ammonia sensor portion 42 detects the ammonia concentration of the gas-to-be-measured whose ammonia component has been decomposed.

Furthermore, in the case of Examples 1, 2 and 5 to 7 in which the gas diffusion holes 8a are disposed within the positional range R₂ along the axial direction between the inner gas discharge hole 143b and the ammonia sensor portion 42, the gas diffusion holes 8a are present on gas-to-be-measured flow paths (flows indicated by the arrows of FIG. 5) extending from the inner gas introduction holes 143a to the inner gas discharge hole 143b. Accordingly, the NOₓ sensor portion 30 can measure NOₓ concentration in such a condition that sufficient gas-to-be-measured is supplied (diffused) thereto. Thus, responsiveness in NOₓ detection improves. As a result, the NOₓ sensor portion 30 can stably measure NOₓ concentration.

Furthermore, in the case of Examples 1 to 4 in which the ammonia sensor portion 42 projects from the gas sensor element 100A, and Examples 5 to 7 in which a portion of the inner protector 143 which faces the ammonia sensor portion 42 is formed into the diameter-reduced portion 143p, by means of the venturi effect, the flow rate of the gas-to-be-measured in a gap (the gas detection chamber 129) between the inner protector 143 and the gas sensor element 100A as measured in the vicinity of the ammonia sensor portion 42 is higher than the flow rate of the gas-to-be-measured in the gap as measured in the other region. Thus, sufficient gas-to-be-measured is supplied (diffused) to the ammonia sensor portion 42, thereby improving accuracy in measuring ammonia concentration.

The invention has been described in detail by reference to the above embodiments. However, the invention should not be construed as being limited thereto. It should further be apparent to those skilled in the art that the various changes in form and detail of the invention as shown and described above may be made. It is intended that such changes be included within the spirit and scope of the claims pended hereto.

This application is based on Japanese Patent Application No. 2010-276707 filed December 13, 2010, incorporated herein by reference in its entirety.

## Claims

1. A multigas sensor (200A) comprising:
a gas sensor element (100A) extending in an axial direction (O) and having an NOₓ sensor portion (30) with a gas diffusion hole (8a) and an ammonia sensor portion (42) at its front end portion (105), the ammonia sensor portion (42) being exposed from an outer surface of the front end portion (105);
a tubular metallic shell (138) holding the gas sensor element (100A) such that a front end portion of the gas sensor element (105) projects from its front end; and
a closed-bottomed tubular protector (141) fixed to a front end portion of the metallic shell (138) and covering the front end portion of the gas sensor element (105), having a front end wall (143t),
the protector (141) having a gas introduction hole (143a) formed in its side wall (143d), and a gas discharge hole (143b) formed in its front end wall (143t) adapted to discharge a gas-to-be-measured;
wherein at least a subportion of the ammonia sensor portion (42) is positioned with respect to the axial direction (O) between the gas introduction hole (143a) and the gas discharge hole (143b),
**characterized in that**
a shortest distance (d1) between the gas introduction hole (143a) and the ammonia sensor portion (42) is shorter than a shortest distance (d2) between the gas introduction hole (143a) and the gas diffusion hole (8a) of the NOₓ sensor portion (30).

2. The multigas sensor (200A) according to claim 1,
wherein the gas diffusion hole (8a) is positioned with respect to the axial direction (O) between the gas discharge hole (143b) and the ammonia sensor portion (42).

3. The multigas sensor (200A) according to claims 1 or 2,
wherein a shortest distance (d3) between the ammonia sensor portion (42) and an inner surface (143s) of the side wall (143d) of the protector (143) is shorter than a shortest distance (d4) between the gas sensor element (100A) excluding the ammonia sensor portion (42) and the inner surface (143s) of the side wall (143d) of the protector (143).

4. The multigas sensor (200A) according to claim 3,
wherein a portion of the side wall (143d) of the protector (143) where a distance between the side wall (143d) and the ammonia sensor portion (42) is shortest projects radially inward.

5. The multigas sensor (200A) according to claims 1 to 4,
wherein the NOₓ sensor portion (30) and the ammonia sensor portion (42) overlap each other in the axial direction (O).

6. The multigas sensor (200A) according to claims 1 to 5,
wherein the shortest distance (d1) between the ammonia sensor portion (42) and the gas introduction hole (143a) is shorter than a shortest distance (d5) between the ammonia sensor portion (42) and the gas discharge hole (143b).

7. The multigas sensor (200A) according to one of claims 1 to 6,
wherein the NOₓ sensor portion (30) comprises:
a first pumping cell (2) having a first solid electrolyte body (2a), and a pair of first electrodes (2b, 2c) disposed on the first solid electrolyte body (2a) so as to be located inside and outside, respectively, a first measuring chamber (S1), and which first pumping cell (2) is adapted to pump oxygen out of or pump oxygen into the gas-to-be-measured that has been introduced into the first measuring chamber (S1) via a gas diffusion hole (8a), and
a second pumping cell (4) having a second solid electrolyte body (4a), and a pair of second electrodes (4b, 4c) disposed on the second solid electrolyte body (4a) so as to be located inside and outside, respectively, an NOₓ measuring chamber (S2) in communication with the first measuring chamber (S1),
wherein a second pumping current flowing between the paired second electrodes (4b, 4c) of the second pumping cell (4) flows in accordance with NOₓ concentration in the gas-to-be-measured whose oxygen concentration has been adjusted in the first measuring chamber (S1) and which has flowed into the NOₓ measuring chamber (S2).

8. The multigas sensor (200A) according to one of claims 1 to 7,
wherein the ammonia sensor portion (42) is configured such that at least a pair of electrodes (42a) is disposed on a solid electrolyte body (25), and which ammonia sensor portion (42) is adapted to output an ammonia concentration output.

## Patentansprüche

1. Multi-Gassensor (200A), der umfasst:
ein Gassensor-Element (100A), das sich in einer axialen Richtung (O) erstreckt und einen NOx-Sensorabschnitt (30) mit einem Gasdiffusions-Loch (8a) sowie einen Ammoniak-Sensorabschnitt (42) an seinem vorderen Endabschnitt (105) aufweist, wobei der Ammoniak-Sensorabschnitt (42) über eine Außenfläche des vorderen Endabschnitts (105) freiliegt;
eine röhrenförmige Metallhülse (138), die das Gassensor-Element (100A) so aufnimmt, dass ein vorderer Endabschnitt des Gassensor-Elementes (105) von ihrem vorderen Ende vorsteht; sowie
eine Schutzeinrichtung (141) in Form einer Röhre mit geschlossenem Boden, die an einem vorderen Endabschnitt der Metallhülse (138) befestigt ist und den vorderen Endabschnitt des Gassensor-Elementes (105) abdeckt, wobei sie eine vordere Abschlusswand (143t) aufweist, und
die Schutzeinrichtung (141) ein Gaseinleit-Loch (143a), das in ihrer Seitenwand (143d) ausgebildet ist, sowie ein Gasausleit-Loch (143b) aufweist, das in ihrer vorderen Abschlusswand (143t) ausgebildet und zum Ausleiten eines zu messenden Gases eingerichtet ist;
wobei wenigstens ein Teilabschnitt des Ammoniak-Sensorabschnitts (42) in Bezug auf die axiale Richtung (O) zwischen dem Gaseinleit-Loch (143a) und dem Gasausleit-Loch (143b) positioniert ist,
**dadurch gekennzeichnet, dass**
ein kürzester Abstand (d1) zwischen dem Gaseinleit-Loch (143a) und dem Ammoniak-Sensorabschnitt (42) kürzer ist als ein kürzester Abstand (d2) zwischen dem Gaseinleit-Loch (143a) und dem Gasdiffusions-Loch (8a) des NOx-Sensorabschnitts (30).

2. Multi-Gassensor (200A) nach Anspruch 1,
wobei das Gasdiffusions-Loch (8a) in Bezug auf die axiale Richtung (O) zwischen dem Gasausleit-Loch (143b) und dem Ammoniak-Sensorabschnitt (42) positioniert ist.

3. Multi-Gassensor (200A) nach Anspruch 1 oder 2,
wobei ein kürzester Abstand (d3) zwischen dem Ammoniak-Sensorabschnitt (42) und einer Innenfläche (143s) der Seitenwand (143d) der Schutzeinrichtung (143) kürzer ist als ein kürzester Abstand (d4) zwischen dem Gassensor-Element (100A) ohne den Ammoniak-Sensorabschnitt (42) und der Innenfläche (143s) der Seitenwand (143d) der Schutzeinrichtung (143).

4. Multi-Gassensor (200A) nach Anspruch 3,
wobei ein Abschnitt der Seitenwand (143d) der Schutzeinrichtung (143), an dem ein Abstand zwischen der Seitenwand (143d) und dem Ammoniak-Sensorabschnitt (42) am kürzesten ist, radial nach innen vorsteht.

5. Multi-Gassensor (200A) nach den Ansprüchen 1 bis 4,
wobei der NOx-Sensorabschnitt (30) und der Ammoniak-Sensorabschnitt (42) einander in der axialen Richtung (O) überlappen.

6. Multi-Gassensor (200A) nach den Ansprüchen 1 bis 5,
wobei der kürzeste Abstand (d1) zwischen dem Ammoniak-Sensorabschnitt (42) und dem Gaseinleit-Loch (143a) kürzer ist als ein kürzester Abstand (d5) zwischen dem Ammoniak-Sensorabschnitt (42) und dem Gasausleit-Loch (143b).

7. Multi-Gassensor (200A) nach einem der Ansprüche 1 bis 6,
wobei der NOx-Sensorabschnitt (30) umfasst:
eine erste Pumpzelle (2), die einen ersten Festelektrolyt-Körper (2a) sowie ein Paar erster Elektroden (2b, 2c) hat, die an dem ersten Festelektrolyt-Körper (2a) so angeordnet sind, dass sie sich innerhalb bzw. außerhalb einer ersten Messkammer (S1) befinden, wobei die erste Pumpzelle (2) so eingerichtet ist, dass sie aus dem zu messenden Gas, das über ein Gasdiffusions-Loch (8a) in die erste Messkammer (S1) eingeleitet worden ist, Sauerstoff herauspumpt oder Sauerstoff in dieses hineinpumpt, sowie
eine zweite Pumpzelle (4), die einen zweiten Festelektrolyt-Körper (4a) und ein Paar zweiter Elektroden (4b, 4c) hat, die in dem zweiten Festelektrolyt-Körper (4a) so angeordnet sind, dass sie sich innerhalb bzw. außerhalb einer NOx-Messkammer (S2) befinden, die mit der ersten Messkammer (S1) in Verbindung steht,
wobei ein zweiter Pump-Strom, der zwischen den paarigen zweiten Elektroden (4b, 4c) der zweiten Pumpzelle (4) fließt, entsprechend NOx-Konzentration in dem zu messenden Gas fließt, dessen Sauerstoffkonzentration in der ersten Messkammer (S1) reguliert worden ist und das in die NOx-Messkammer (S2) geströmt ist.

8. Multi-Gassensor (200A) nach einem der Ansprüche 1 bis 7,
wobei der Ammoniak-Sensorabschnitt (42) so ausgeführt ist, dass wenigstens ein Paar Elektroden (42a) an dem Festelektrolyt-Körper (25) angeordnet ist, und der Ammoniak-Sensorabschnitt (42) so eingerichtet ist, dass er einen Ammoniakkonzentrations-Ausgang ausgibt.

## Revendications

1. Capteur multigaz (200A) comprenant :
un élément capteur de gaz (100A) qui s'étend en direction axiale (O) et comporte une portion à capteur de NOₓ (30) avec un trou de diffusion de gaz (8a) et une portion à capteur d'ammoniac (42) sur sa portion d'extrémité avant (105), la portion à capteur d'ammoniac (42) étant exposée depuis une surface externe de la portion d'extrémité avant (105) ;
une coque métallique tubulaire (138) qui maintient l'élément capteur de gaz (100A) de telle sorte qu'une portion d'extrémité avant de l'élément capteur de gaz (105) se projette depuis son extrémité avant ; et
un protecteur tubulaire à fond fermé (141) fixé à une portion d'extrémité avant de la coque métallique (138) et recouvrant la portion d'extrémité avant de l'élément capteur de gaz (105), comportant une paroi d'extrémité avant (143t),
le protecteur (141) comportant un trou d'introduction de gaz (143a) formé sur sa paroi latérale (143d), et un trou d'évacuation de gaz (143b) formé dans sa paroi d'extrémité avant (143t) adapté pour évacuer un gaz à mesurer ;
dans lequel au moins une sous-portion de la portion à capteur d'ammoniac (42) est positionnée par rapport à la direction axiale (O) entre le trou d'introduction de gaz (143a) et le trou d'évacuation de gaz (143b),
**caractérisé en ce que**
la distance la plus courte (d1) entre le trou d'introduction de gaz (143a) et la portion à capteur d'ammoniac (42) est inférieure à la distance la plus courte (d2) entre le trou d'introduction de gaz (143a) et le trou de diffusion de gaz (8a) de la portion à capteur de NOₓ (30).

2. Capteur multigaz (200A) selon la revendication 1,
dans lequel le trou de diffusion de gaz (8a) est positionné par rapport à la direction axiale (O) entre le trou d'évacuation de gaz (143b) et la portion à capteur d'ammoniac (42).

3. Capteur multigaz (200A) selon la revendication 1 ou 2,
dans lequel la distance la plus courte (d3) entre la portion à capteur d'ammoniac (42) et une surface interne (143s) de la paroi latérale (143d) du protecteur (143) est inférieure à la distance la plus courte (d4) entre l'élément capteur de gaz (100A), en excluant la portion de capteur d'ammoniac (42), et la surface interne (143s) de la paroi latérale (143d) du protecteur (143).

4. Capteur multigaz (200A) selon la revendication 3,
dans lequel une portion de la paroi latérale (143d) du protecteur (143) où la distance entre la paroi latérale (143d) et la portion à capteur d'ammoniac (42) est la plus courte se projette radialement vers l'intérieur.

5. Capteur multigaz (200A) selon les revendications 1 à 4,
dans lequel la portion à capteur de NOₓ (30) et la portion à capteur d'ammoniac (42) se chevauchent en direction axiale (O).

6. Capteur multigaz (200A) selon les revendications 1 à 5,
dans lequel la distance la plus courte (d1) entre la portion à capteur d'ammoniac (42) et le trou d'introduction de gaz (143a) est inférieure à la distance la plus courte (d5) entre la portion à capteur d'ammoniac (42) et le trou d'évacuation de gaz (143b).

7. Capteur multigaz (200A) selon l'une des revendications 1 à 6,
dans lequel la portion à capteur de NOₓ (30) comprend :
une première cellule de pompage (2) ayant un premier corps d'électrolyte solide (2a), et une paire de premières électrodes (2b, 2c) disposée sur le premier corps d'électrolyte solide (2a) de manière à se trouver respectivement à l'intérieur et l'extérieur d'une première chambre de mesure (S1), et ladite première cellule de pompage (2) étant adaptée pour pomper de l'oxygène hors du gaz à mesurer ou dans le gaz à mesurer, qui a été introduit dans la première chambre de mesure (S1) via un trou de diffusion de gaz (8a), et
une deuxième cellule de pompage (4) ayant un deuxième corps d'électrolyte solide (4a), et une paire de deuxièmes électrodes (4b, 4c) disposée sur le deuxième corps d'électrolyte solide (4a) de manière à se trouver respectivement à l'intérieur et l'extérieur d'une chambre de mesure de NOₓ (S2) en communication avec la première chambre de mesure (S1),
dans lequel un deuxième courant de pompage qui s'écoule entre la paire de deuxièmes électrodes (4b, 4c) de la deuxième cellule de pompage (4) s'écoule conformément à la concentration en NOₓ dans le gaz à mesurer dont la concentration d'oxygène a été ajustée dans la première chambre de mesure (S1) et qui s'est écoulé dans la chambre de mesure de NOₓ (S2).

8. Capteur multigaz (200A) selon l'une des revendications 1 à 7,
dans lequel la portion à capteur d'ammoniac (42) est configurée de telle sorte qu'au moins une paire d'électrodes (42a) est disposée sur un corps d'électrolyte solide (25), et ladite portion à capteur d'ammoniac (42) est adaptée pour sortir une sortie de concentration d'ammoniac.
